Europäisches Patentamt

European Patent Office

Office européen des brevets

(19)

(11) Publication number: **0 359 880**
**A1**

## EUROPEAN PATENT APPLICATION
(12)

(21) Application number: **88308792.6**

(22) Date of filing: **22.09.88**

(51) Int. Cl.⁵: **A61M 16/08**

(43) Date of publication of application:
**28.03.90 Bulletin 90/13**

(84) Designated Contracting States:
**DE GB**

(71) Applicant: **Linder, Gerald Seymour**
**16693 Charmel Lane**
**Pacific Palisades, CA 90272(US)**

(72) Inventor: **Linder, Gerald Seymour**
**16693 Charmel Lane**
**Pacific Palisades, CA 90272(US)**

(74) Representative: **Williams, Trevor John et al**
**J.A. KEMP & CO. 14 South Square Gray's Inn**
**London WC1R 5EU(GB)**

(54) **Breathing circuit connector for use in anaesthesiology.**

(57) A breathing circuit connector (31) for intercoupling between the flexible hoses of an anaesthesia machine and the input connector of an endotracheal tube comprises a hollow body of relatively rigid material having a cylindrical male input section (32) and a cylindrical female output section (36). A cylindrical bore (40) within the hollow body extends between the cylindrical input (32) and output sections (36). The bore within the cylindrical output (36) section consists of a series of two or more cylindrical portions (37,38,39) having different internal diameters. The internal diameter of the first bore portion (37) adjacent the output end of the cylindrical output section (36) is slightly larger than the internal diameter of the bore portion situated within the cylindrical output section. The breathing circuit connector may be formed as an elbow with the longtiduinal axis of the bore of the cylindrical output section being inclined relative to the longitudinal axis of the bore of the cylindrical input section. Alternatively, the improved connector may comprise a single female output section and a pair of cylindrical male sections, each section having a bore whose axis is displaced from the other two to form a Y-shaped connector.

FIG. 3

# BREATHING CIRCUIT CONNECTOR FOR USE IN ANAESTHESIOLOGY

The present invention relates to connectors, and, in particular, to an improved breathing circuit connector for intercoupling between the flexible hoses of an anaesthesiology machine and the input of an endotracheal tube connector.

The convention breathing circuit connector consists of a hollow cylindrical body of rigid or semi-rigid plastic material having a straight or tapered male input section, a normally straight female output section, and a cylindrical bore extending between the input and output sections.

The connector may be shaped as an elbow with the longitudinal axis of the bore portion within the input section displaced 90° from the longitudinal axis of the bore portion within the output section.

Alternatively, the bore may extend in a straight line or it may be curved.

Another type of breathing circuit connector consists of a single female output section and a pair of male cylindrical sections displaced from each other, the connector being in the shape of a Y. The output female section of the conventional cylindrical breathing circuit connector has a bore of standard, uniform diameter, i.e. fifteen millimeters, and is adapted for coupling to the male input section of an endotracheal tube connector. The male input section of the breathing circuit connector is adapted for insertion either into the flexible, corrugated hose from an anaesthesiology machine or for insertion into the output female section of the conventional Y-shaped connector. The two male sections of the conventional Y-shaped connector are securely attached, respectively, to two separate and independent flexible hoses from the anaesthesiology machine, as by cementing.

Prior art breathing circuit connectors have been found to possess a rather serious problem in attempting to maintain a secure, air-tight coupling between a tapered male input section and a corresponding female output section. When the material forming the male input section is composed of a relatively rigid or semi-rigid plastic or polymer material, such as nylon, polyethylene or polypropylene, a rather strong force is needed to ensure that the male input section is firmly inserted into the female output section. Where the female output section has either a straight, uniform, cylindrical internal diameter, or a tapered internal diameter, the coupling between the male and female sections has been found to work loose and, at times, to become completely disconnected. This has been especially true when the material forming the female output sction is also relatively hard or semi-rigid. The failure of the breathing circuit connection, either during an operation or in post-operative recovery, represents a very serious hazard to the health of the patient. The same danger exists where the female output section of the conventional elbow, or 90° breathing circuit connector, is coupled to a tapered male input section of the conventional, hard plastic, endotracheal tube connector. The problems also exist between the coupling between the female output section of the conventional Y-shaped breathing circuit connector and the male input section of the conventional endotracheal tube connector.

One solution to the above problem has been to employ bands, cords or adhesive to bind together each connection in the breathing circuit coupling. To facilitate the use of cords or bands, a crown frequently is formed atop the hard plastic breathing circuit connector to serve as a base or anchor to which the bands, cords or adhesive may be attached.

Another solution has been to employ breathing circuit connectors formed of resilient material having a somewhat higher coefficient of friction in the hope that the required air-tight coupling will be maintained. With this type of connector, it is very difficult to disconnect the coupling, as is often required during an operation.

Still another solution has been to employ breathing circuit connectors designed to include an external ridge or ring upon the outside surface of the male input section in order to engage a mating internal groove formed inside the female section of the connector, as disclosed in U.S. Patents 3,552,778 and 4,152,017.

A partial solution to the above problem has been the introduction of an improved endotracheal tube connector having a male input section consisting of a series of three external stepped diameter portions, as described in my U.S. Patent 4,369,911. This particular endotracheal tube connector has been found to maintain a secure and reliable air-tight coupling to the female output section of the standard fifteen millimeter breathing circuit connector.

A principal object of the present invention is to provide a breathing circuit connector having a more secure and reliable air-tight coupling to an endotracheal tube connector.

The present invention overcomes the above problems by the elimination of the tapered bore, or the straight bore, of the female output section of the conventional breathing circuit connector. This is achieved by designing the internal bore in the form of a series of straight and uniform internal diameter bore portions of slightly different diameters. This design feature enables one or more of the internal bore portions to seat upon the male input section of the endotracheal tube connector or upon the male input section of the conventional elbow breathing circuit connector in such manner as to reduce the internal forces that normally cause the coupling to separate and, thereby, disconnect.

The invention also makes it possible to provide a breathing circuit connector which is easier to connect to, and to disconnect form, an endotracheal tube connector; it is furthermore suitable for use with endotracheal tube connectors of different manufacture, and is capable of achieving an airtight coupling despite small dimensional variations in the material forming the connector.

The present invention will be more fully understood, and further objects and advantages will become apparent, from a study of the following detailed description in connection with the drawings, in which:

Fig. 1 is a side view of a prior art endotracheal tube connector adapted for insertion into the open proximal end of a conventional endotracheal tube;

Fig. 2 is a side view of one prior art type of breathing circuit connector for intercoupling between the flexible hoses of an anaesthesiology machine and the input to an endotracheal tube connector;

Fig. 3 is a side view of the improved breathing circuit connector of the present invention;

Fig. 4 is a side view of an alternative breathing circuit connector of the present invention;

Fig. 5 is a side view of an improved endotracheal tube connector attached to the open proximal end of an endotracheal tube, as disclosed in my U.S. Patent 4,369,991.

Fig. 6 is a side view of an alternative Y-type brething circuit connector in accordance with the present invention.

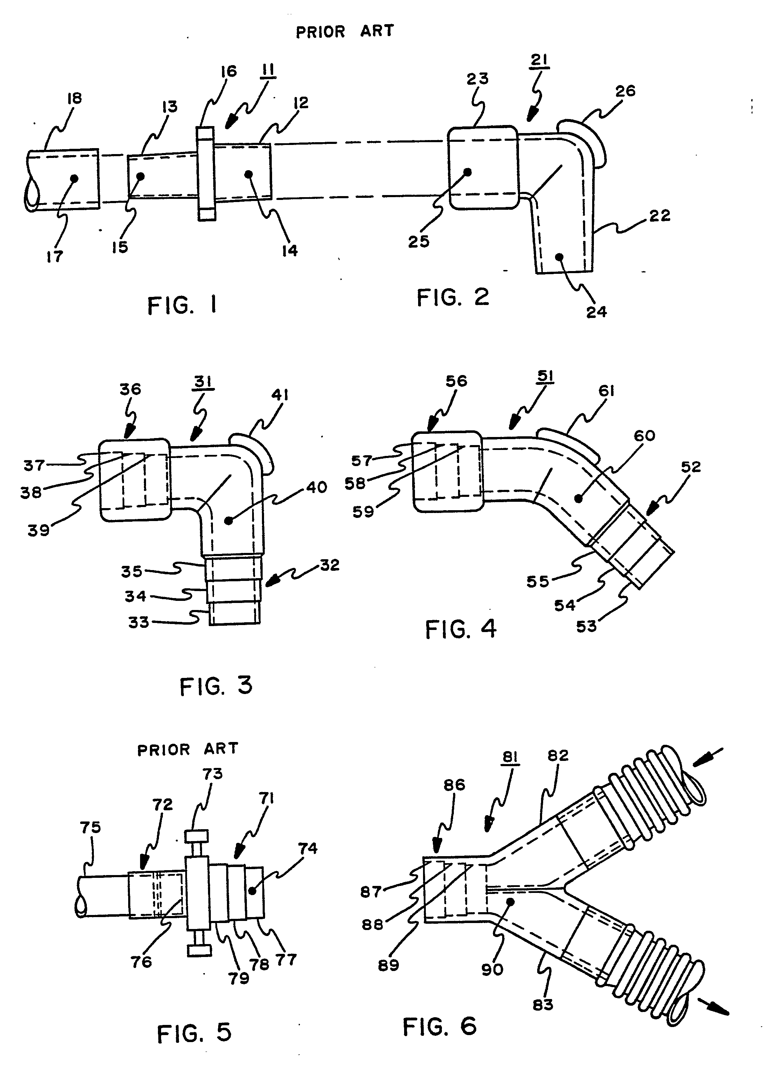

Fig. 1 illustrates one type of prior art endotracheal tube connector 11 having a hollow, externally tapered cylindrical input section 12, a hollow externally tapered cylindrical output section 13 and a bore 14, 15 extending therebetween. Connector 11 employs a centrally located external flange portion 16 between the input and output sections 12 and 13 to enable the using physician to hold the connector 11 between the thumb and fingers. The output section 13 is adapted for insertion into the open proximal end 17 of a conventional endotracheal tube 18. The size of output section 13 and bore 15 may vary to accommodate different sizes of endotracheal tubes.

Fig. 2 illustrates one type of prior art breathing circuit connector 21 of the elbow, or 90°, type. Connector 21 usually is composed of a relatively hard plastic material, such as polyethylene, polypropylene, or nylon and includes a hollow externally tapered male input section 22, a hollow cylindrical female output section 23, and a bore 24, 25 extending therebetween. A crown 26 is integrally formed upon the top outer surface of connector 21, as shown. The internal diameter of bore 25 of the female output section 23 is of standard, uniform size, namely fifteen millimeters.

Fig. 3 illustrates one embodiment of the improved breathing circuit connector 31 of the present invention. Connector 31 includes a hollow, cylindrical male input section 32 hving a series of three different outer diameter portions 33, 34 and 35; a hollow, cylindrical female output section 36, having a series of three different internal diameter or bore portions 37, 38, 39 and a bore 40 extending between the bore portion 39 and the open end of male input section 32. A crown 41 formed integrally with the connector appears at the outer apex of the connector.

Each of the outer diameter portions 33, 34 and 35 of male input section 32 is of uniform diameter and they differ, slightly, from each other. The first or outermost diameter portion 33 adjacent the input end is slightly smaller than the third or innermost diameter portion 35; the second or intermediate diameter portion 34 is of larger diameter than first portion 33 and of smaller diameter than the third or innermost portion 34. The male input section 32, with its series of outer diameter portions 33, 34 and 35, is substantially identical to the male input section of the improved endotracheal tube connector of my above-mentioned U.S. Patent 4,369,991.

Each of the series of three different internal diameter or bore portions 37, 38 and 39 of output section 36 are similarly of uniform diameter and differ slightly from each other by a relatively small amount, i.e., in the neighborhood of two- to four-thousandths of an inch, or about .05 to .1 millimeter. The series of internal bore portions 37, 38 and 39 differ one from the other by similar amounts. It will be appreciated, therefore, that the illustrations of these portions in Fig. 3 are not necessarily drawn to scale and are, in fact, shown to be larger than these relatively small amounts for the purpose of illustration.

The embodiment of the invention illustrated in Fig. 4 shows the improved breathing circuit connection 51 having a hollow, cylindrical male input section 52, a hollow, cylindrical female output section 56, and a bore 60 extending therebetween. A series of three different outer diameter portions 53, 54 and 55 appear upon the outer cylindrical surface of male input section 52. A series of three different internal diameter portion 57, 58 and 59 appear within the inner cylindrical surface of female output section 56. Crown 61 is formed integrally upon the outer surface of the connector, as shown.

The dimensions of the outer diameter portions 53, 54 and 55 upon the outer cylindrical surface of male input section 52 correspond to those of the outer diameter portions 33, 34 and 35 of connector 31 of Fig. 3.

Similarly, the dimensions of the internal diameter portions 57, 58 and 59 correspond to those of the internal diameter portions 37, 38 and 39 of connector 31 of Fig. 3.

The endotracheal tube connector of my above-mentioned U.S. Patent 4,369,911, illustrated in Fig. 5, consists of a hollow, cylindrical input section 71, a hollow, cylindrical output section 72, a central flange portion 73 and a bore 74 extending therethrough. The outer cylindrical surface of input section 71 is stepped in the same manner as the section 32 of Figure 3 described above.

The cylindrical output section 72 is shown installed over the open proximal end 76 of a conventional endotracheal tube 75, as described in U.S. Patent 4,369,911.

The embodiment of the invention of Fig. 6 shows a breathing circuit connector 81 having a pair of hollow, cylindrical male sections 82 and 83 in the shape of a Y. The upper section 82 is securely coupled, as by cementing, to a corrugated or flexible hose from the anaesthesiology machine. This upper section 82 receives the anaesthetizing gas, or oxygen, as the cases may be, from the anaesthesiology machine. The lower section 83, securely coupled to a second corrugated, flexible hose, returns the exhaled gases from the patient back to the anaesthesiology machine. Both male sections 82 and 83 are smooth, untapered and of uniform external diameter.

A single, hollow female output sectin 86 includes a series of three different internal diameter or bore portions 87, 88 and 89. A bore 90 extends between the bore portion 89 and the pair of hollow, cylindrical male sections 82 and 83. The dimensions of the internal diameter portions 87, 88 and 89 correspond to those of the hollow, female output sections 36 and 56 of the connectors 31 and 51 of Figs. 3 and 4.

The hollow, female output sections 36 and 56, of the connectors 31 and 51 of Figs. 3 and 4, are adapted for a secure air-tight coupling to the male input section 12 of the conventional endotracheal tube connector 11, of Fig. 1, or to the male input section 71 of the improved endotracheal tube connector of Fig. 5. It is preferred, however, that the improved breathing circuit connectors of the present invention be used with the improved endotracheal tube connector of Fig. 5.

The hollow, female output section 86, of the connector 81 of Fig. 6, is adapted for a secure and air-tight coupling to the male input section 22 of the conventional breathing circuit connector 21 of Fig. 2, or to the male input sections 32 and 52 of the connectors 31 or 51 of Figs. 3 or 4. The hollow, female output section 86 of the connector 81 of Fig. 6 is also adapted for a secure, air-tight coupling directly to the hollow input section 71 of the improved endotracheal tube connector of Fig. 5.

An improved coupling is achieved between the female output sections 36, 56 and 86 of the brething circuit connectos of the present invention and the male input sections of endotracheal tube connectors by virtue of the series of different internal diameter portions located within the hollow, female output sections. This improved coupling also exists between the female output section of the connector of Fig. 6 and the male input section 32 or 52 of the connectors 31 or 51 of Figs. 3 and 4. Since each of the internal diameter portions is uniform and untapered, and since each internal diameter portion differs slightly inb diameter from its adjacent portion, a smooth and air-tight coupling can be achieved between at least one of the internal diameter portions with one of the smooth and untapered diameter portions of the male input section. for example, referring to Figs. 5 and 6, the internal diameter portion 87 of female output section 86 may achieve a smooth and snug fit upon either the external diameter portion 77, or upon external diameter portion 78, or upon external diameter portion 79, depending, of course, upon the relative differences in the respective diameters of these diameter portions. Additionally, the internal diameter portion 88 of output section 86 may be found to fit smoothly upon external diameter portion 78. Finally, it may be found that internal diameter portion 89 of output section 86 will fit upon the external diameter portion 77, depending, of course, upon the relative diameters of these mating portions. Accordingly, it is noted that the diameters of each of the portions 87, 88 and 89 do not necessarily have to correspond to the diameters of portions 79, 78 and 77. It has been found that a snug and tight fit of any one of the internal diameter portions of the female output section with any one of the extenal diameter portions of the mating male input section achieves a secure and air-tight coupling. Since each of the mating diameter portions is uniform and untapered, the forces normally found to exist in the conventional tapered sections tending to cause separation of the coupling do not exist in the improved connectors of the present invention. The improved connectors are generally molded of relatively hard plastic material, and by virtue of the several different diameter portions are capable of achieving an airtight coupling despite small variations in manufacturing tolerances which are likely to occur during manufacture.

Claims

1. A breathing circuit connector for coupling the output of an anaesthesiology machine to the input connector of an endotracheal tube, comprising a hollow body of relatively rigid material having a cylindrical input section adapted for coupling to the output of an anesthesiology machine, said hollow body further having a cylindrical output section adapted for coupling to the open input end of an endotracheal tube; a cylindrical bore being situated within and extending between the cylindrical input and output sections of said hollow body, the bore within the cylindrical output section of said hollow body consisting of a series of cylindrical portions of different diameters, the diameter of the first bore portion situated adjacent the output end of the cylindrical output section being slighlty larger than the diameter of the bore portion internal of said cylindrical output section.

2. A breathing circuit connector as claimed in claim 1, wherein the bore within the cylindrical output section of said hollow body consists of a series of three cylindrical portions of different diameters, the diameter of the first bore portion situated adjacent the output end of the cylindrical output section being larger than the diameter of the third or innemost bore portion, the diameter of the second or intermediate bore portion being less than the diameter of the first bore portion and larger than the diameter of the third or innermost bore portion.

3. A breathing circuit connector as claimed in claim 1 or 2, wherein the longitudinal axis of the bore portions within the cylindrical output section of said hollow body is non-concentric with the longitudinal axis of the bore portion within the cylindrical input section of said hollow body.

4. A breathing circuit connector and claimed in claim 1 or 2, wherein the longtiduinal axis of the bore portions within the cylindrical output section of said hollow body is different from the longtiduinal axis of the bore portion within the cylindrical input section of said hollow body.

5. A breathing circuit connector as claimed in any preceding claim, wherein said hollow body of relatively rigid material has an input section consisting of a pair of hollow cylindrical portions angularly displaced one with respect to the other in the shape of a Y, the longitudinal axis of each of said pair of hollow cylindrical portions being angularly displaced with respect to the longitudinal axis of the bore portions within the cylindrical output section of said hollow body.

PRIOR ART

FIG. 1

FIG. 2

FIG. 3

FIG. 4

PRIOR ART

FIG. 5

FIG. 6

European Patent Office

# PARTIAL EUROPEAN SEARCH REPORT

which under Rule 45 of the European Patent Convention shall be considered, for the purposes of subsequent proceedings, as the European search report

Application number

EP 88 30 8792

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | US-A-4 369 991 (LINDER)<br><br>* Column 4, line 57 - column 5, line 5; column 5, lines 34-45; column 7, lines 36-53; figures 3,6 *<br><br>-- | 1,2,4,5 | A 61 M 16/08 |
| Y | US-A-4 569 344 (PALMER)<br><br>* Column 10, line 65 - column 11, line 15; column 11, lines 33-37; figures 3,8,9 *<br><br>-- | 1,2,4,5 | |
| Y | WO-A-81 02 675 (SONTEK)<br><br>* Figures 6,7,10 *<br><br>-- | 4,5 | |
| A | FR-A-2 522 969 (TERUMO)<br><br>* Page 15, line 33 - page 16, line 29; figures 8,9 *<br><br>-- <br><br>./. | 1 | TECHNICAL FIELDS SEARCHED (Int. Cl.4)<br><br>A 61 M<br>F 16 L |

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1,2,4,5
Claims searched incompletely:
Claims not searched: 3
Reason for the limitation of the search:

In claim 3 the meaning of "non-concentric arrangement of the two longitudinal axes" is not clear

| Place of search | Date of completion of the search | Examiner |
|---|---|---|
| The Hague | 24-04-1989 | SCHÖNLEBEN |

EPO Form 1505.1 03.82

| Category | Citation of document with indication, where appropriate, of relevant passages | Relevant to claim |
|---|---|---|
| | **DOCUMENTS CONSIDERED TO BE RELEVANT** | |
| A | US-A-3 918 450 (BHUPENDRA)<br>* Figure 1 *<br><br>-- | 2 |
| E | US-A-4 774 940 (LINDER)<br>* Whole document *<br><br>-------- | 1,2,4,<br>5 |

**CLASSIFICATION OF THE APPLICATION (Int. Cl.4)**

**TECHNICAL FIELDS SEARCHED (Int Cl.4)**

EPO Form 1505.3 06.78